# EUROPÄISCHE PATENTSCHRIFT

(11) **EP 2 505 161 B1**
(45) Veröffentlichungstag und Bekanntmachung des Hinweises auf die Patenterteilung: **27.07.2016**
(21) Anmeldenummer: 12161417.6
(22) Anmeldetag: 27.03.2012
(51) Int. Cl.: A61B 90/57, A61G 13/10, A61B 17/02

(54) **Klemmvorrichtung mit Kniehebel zur Befestigung an der Profilschiene eines Operationstisches**
Clamping system with a toggle lever for the attachment to the side-rail of an operating table
Dispositif de fixation à levier coudé pour rail lateral d'une table d'opération

(30) Priorität: 31.03.2011 DE 102011001694; 09.11.2011 DE 102011055158
(43) Veröffentlichungstag der Anmeldung: 03.10.2012
(73) Patentinhaber: Unger, Michael, 78573 Wurmlingen (DE)
(72) Erfinder: Unger, Michael, 78573 Wurmlingen (DE); Heiter, Uwe, 78056 VS-Schwenningen (DE); Soult, Hendrik, 78126 Königsfeld (DE)
(74) Vertreter: Patentanwälte und Rechtsanwalt Weiß, Arat & Partner mbB

(56) Entgegenhaltungen:
- WO-A1-2006/085091
- DE-A1- 3 149 215
- US-A1- 2007 213 597
- US-A1- 2009 308 400
- US-A1- 2010 299 890

## Beschreibung

Die Erfindung betrifft eine Vorrichtung zur Befestigung an einem Operationstisch nach dem Oberbegriff des Anspruchs 1.

### Stand der Technik

Aus dem Stand der Technik sind vielfältige Vorrichtungen zur Befestigung von Operationshilfen an einem Operationstisch bekannt. Nachteilig an diesen Vorrichtungen ist der Umstand, dass diese gerade bei schweren Operationshilfen nicht derart fest mit dem Operationstisch verbunden werden können, als dass ein Verrutschen ausgeschlossen wäre, so kann es gerade während einer Operation zu einem Verrutschen oder Lösen der Vorrichtungen kommen, was wiederum eine Gefährdung des Patienten bedeutet.

In diesem Zusammenhang wird auf die US 2007/213597 A1 hingewiesen. Dort wird eine Vorrichtung zur Befestigung eines Grundelements an einem Operationstisch beschrieben. Weiter wird auf die DE 31 49 215 A1 hingewiesen. Dort wird eine Befestigungskloben offenbart, welcher für die Festlegung an einem Linearprofil vorgesehen ist. Daneben wird auf die US 2009/308400 A1 und die WO 2006/085091 A1 verwiesen, welche ebenfalls Vorrichtungen zur Befestigung von medizinischen Geräten an einem Operationstisch zeigen.

Die WO 2006/085091 wird dabei als nächstliegender Stand der Technik gegenüber dem Gegenstand des Anspruchs 1 betrachtet

### Aufgabe

Aufgabe der Erfindung ist es eine Vorrichtung zu schaffen, welche eine feste Verbindung mit dem Operationstisch gewährleistet und ausserdem in der Lage ist verschiedene Arten von Operationshilfen aufzunehmen.

### Lösung der Aufgabe

Zur Lösung der Aufgabe führt der kennzeichnende Teil des Patentanspruchs 1.

Eine Vorrichtung zur Befestigung an einem Operationstisch dient in erster Linie dazu, eine möglichst sichere Verbindung mit dem Operationstisch oder Teilen des Operationstisches zu gewährleisten.

Als Teile der Operationstische sollen insbesondere herkömmlicher Weise angebrachten Haltegriffe oder Relinge oder Ausformungen gelten. Diese Teile stellen sich in der Regel als längliche Profile dar, welche im wesentlichen über die gesamte Länge des Operationstisches reichen. Die Profile sind regelmässig rechteckig gestaltet, wobei die Standardmasse 10 x 25 mm betragen. Die Profile dienen dem Krankenhauspersonal bspw. als Haltegriffe, um den Operationstisch zu verschieben. Die Profile können aber auch zur Befestigung von Operationshilfen genutzt werden. Allerdings gestaltet sich das an den Kanten leicht abgeflachte Profil als eine unzureichende Befestigungsgrundlage für herkömmliche Vorrichtungen zur Befestigung einer Operationshilfe an dem Operationstisch. Vorteilhafterweise ist der erfindungsgemässen Vorrichtung gerade eine auf das im Schnitt rechteckige Profil zugeschnittene Lösung gegeben, welche einen festen Sitz der Vorrichtung an dem Profil gewährleistet.

Ein Operationstisch im Sinne der Erfindung soll unabhängig von Material oder unterschiedlicher nationaler Standardnormen jede Konstruktion sein, welche geeignet ist, für eine Operation am menschlichen Körper genutzt zu werden. Herkömmlicherweise handelt es sich hierbei um Edelstahltische, welche eben ein oben angesprochenes Profil ausbilden.

Eine Operationshilfe im Sinne der Erfindung soll immer dann gegeben sein, wenn ein operierender Nutzer, bspw. ein Chirurg, zur Übernahme von Kontroll- oder Tätigkeitsaufgaben eine Hilfe in Anspruch nimmt. Dies können bspw. sogenannte Operationsroboter, Überwachungsgeräte, Monitore, Medizinabgabegeräte oder praktische Unterstützung leistende Vorrichtungen sein. Diese Unterstützung leistenden Vorrichtungen werden in der Regel derart gestaltet, dass bspw. vom operierenden Nutzer gesetzte Schnitte gespreizt und offengehalten werden können. Die aufgezeigte Liste von Operationshilfen ist nicht als abschliessende Aufzählung anzusehen.

Die erfindungsgemässe Vorrichtung weist ausserdem ein Grundelement auf. Dieses Grundelement dient dazu, auf das Profil des Operationstisches aufgesetzt zu werden, um weitere Schritte der Befestigung schnell und einfach durchführen zu können. Dazu weist das Grundelement eine entsprechende Ausnehmung auf, welche gerade geeignet ist, die mit 10 mm Kante kürzere Seite des Profils aufzunehmen. Die Ausnehmung weist ausserdem eine Toleranz von 1 bis 3 mm bevorzugt 1 bis 2 mm auf. Diese Toleranz dient dazu, dass ein über das Profil gelegtes steriles Operationstuch bei einem Befestigungsvorgang ebenfalls Platz in der Ausnehmung des Grundteils findet. Vorteilhaft hierbei ist, dass das Grundelement einfach sterilisierbar ist und ausserdem als Basis oder als Widerlager für die übrigen technischen Merkmale der Erfindung dient. Das Grundelement ist derart gestaltet, dass der Nutzer nach dem Aufsetzen des Grundelementes auf das Profil des Operationstisches ohne weiteres mit einer Hand die Vorrichtung an dem Profil halten kann. Dies kann im Einzelnen bedeuten, dass das Grundelemente eine gewisse Masse aufweist, welche den Schwerpunkt nahe des Profils des Operationstisches versetzt. Es kann aber auch bedeuten, dass aufgrund der Formgebung des Grundelements ein entscheidender Bereich des Profils des Operationstisches teilweise umschlossen wird, so dass ein Abrutschen nicht mehr ohne Weiteres erfolgen kann.

Das erfindungsgemässe Grundelement weist ausserdem eine Zuordnung auf. Diese Zuordnung ist vorzugsweise fest mit dem Grundelement verbunden.

In typischen Ausführungsbeispiel ist die Zuordnung austauschbar. Vorzugsweise erfolgt eine Verbindung zwischen der Zuordnung und dem Grundelement über eine Schraub-, Steck- und/oder Klemmverbindung. Dadurch ergibt sich der Vorteil, dass je nach Anwendungszweck eine Zuordnung mit der passenden Länge verwendet werden kann.

Vorteilhaft an der Zuordnung ist der Umstand, dass es bspw. leicht sterilisierbar ist und ausserdem der Aufnahme einer schier unbegrenzten Vielfalt von Operationshilfen dient. Hierbei soll unbeachtlich sein, ob die Zuordnung ein Hohlprofil oder Vollprofil ist. Eine entsprechende Wahl zwischen Hohlprofil und Vollprofil richtet sich ausschliesslich nach den Bedürfnissen des Nutzers oder des Herstellers und ist für die Umsetzung der erfinderischen Idee nicht von Bedeutung. Ein weiterer Vorteil der Zuordnung besteht darin, dass während des Befestigungsvorgangs der Vorrichtung der Nutzer ohne grösseren Aufwand oder Krafteinsatz nur mit Hilfe der Zuordnung das Grundelement aus dem Profil des Operationstisches halten, verschieben oder einfacher befestigen kann.

Eine erfindungsgemässe Vorrichtung weist ausserdem ein Festlegeteil auf. Dieses Festlegeteil dient dazu, dass im Zusammenspiel mit dem Grundelement die Vorrichtung an dem Profil des Operationstisches festgelegt werden kann. Vorteilhaft an dem Festlegeteil ist ein einfacher Aufbau, sowie eine vereinfachte Sterilisierung, da zwischen dem Grundteil und dem Festlegeteil zumindest während der Sterilisierung immer ausreichende Räume bspw. zum Durchspülen vorhanden sind.

Blei der erfindungsgemässen Vorrichtung wird der Befestigungsvorgang dadurch erreicht, dass ein Kniehebelverschluss vorgesehen ist. Dieser Kniehebelverschluss ist derart gestaltet, dass der Nutzer bei Überwinden einer erforderlichen Kraftspitze anschliessend durch die Konstruktion des Kniehebels einen festen Verschluss derart schafft, als das das Grundelement und das Festlegeteil nur durch eine Rücküberwindung der angesprochenen Kraftspitze voneinander gelöst werden können. Ein Verrutschen der Vorrichtung an dem Profil des Operationstisches ist ausgeschlossen, da eine solche Kraftspitze durch bspw. Aufsetzen einer Operationshilfe nicht erreicht werden kann. Lediglich eine bewusste Überwindung dieser erforderlichen Kraftspitze durch einen Nutzer ermöglicht ein Festlegen oder Lösen der erfindungsgemässen Vorrichtung vom Profil des Operationstisches. Hierbei wird ein bekanntes Prinzip, dass Kniehebelelement-Prinzip genutzt, um eine sichere Befestigung der Vorrichtung an dem Profil des Operationstisches vorzunehmen.

Ein erfindungsgemässes Ausführungsbeispiel des Kniehebelverschlusses umfasst eine Federkonstruktion. Diese Federkonstruktion dient dem Toleranzausgleich beim Befestigen. Dies bedeutet im Einzelnen, dass je nach dem, ob ein oder zwei oder auch drei Lagen des Operationstuchs über das Profil des Operationstisches gelegt wurden, der Kniehebelverschluss trotzdem vollständig schliesst. Die entsprechende Vergrösserung des Umfangs des Profils durch die sterilen Operationstücher, welche um das Profil gelegt werden, kann mit der Toleranz der Federkonstruktion aufgefangen werden. Dazu schafft die Federkonstruktion einseitig einen Spielraum und anderseitig einen immer gleich bleibenden Anschlag für den geschlossenen Kniehebelverschluss. Als Federkonstruktion kommen nicht lediglich Federn, sondern jegliche Art von sogenannten Kraftspeichern zum Einsatz. Beispielsweise kann auch ein entsprechender Gummipuffer mit eingesetzt werden, solange er die gleichen Toleranzausgleicheigenschaften wie eine herkömmliche Feder aufweist. Vorteilhaft hierbei ist der Umstand, dass auch bei unvorhergesehenen störenden Elementen oder geringfügig anders gestalteten Profilen unterschiedlicher Operationstische ein festes Anliegen der erfindungsgemässen Vorrichtung durch den Kniehebelverschluss gewährleistet ist.

In einem anderen bevorzugten Ausführungsbeispiel weist die Federkonstruktion ein vorgespanntes Federelement auf. Dieses vorgespannte Federelement beinhaltet den Vorteil, dass unvorhergesehene Änderungen im Rahmen des Profils aufgrund der vorgespannten Lage sofort aufgenommen werden und trotzdem andererseits dem Anschlagpunkts des Kniehebelverschlusses aufgrund des vorgespannten Federelements eine gleichbleibende zu überwindende Kraftspitze erhalten bleibt. Dies gibt dem Nutzer ein Sicherheitsgefühl, dass er nicht den Eindruck bekommt, er müsse aufgrund der aufgelegten Operationstücher mehr Kraft einsetzen und anschliessend befürchten, dass der Kniehebelverschluss bspw. durch die aufgelegten Operationstücher zurückschnappt und die Befestigung vom Profil des Operationstisches gleitet.

Das vorgespannte Federelement ist zwischen einem ersten und einem zweiten Lager angeordnet. Das erste Lager stellt sich als schwenkbares Verbindungsstück zum Festlegeteil dar. Hierbei ist vor allem hervorzuheben, dass das Lager teil - schwenkbar gestaltet sein muss, um den Ablauf des Kniehebelverschlusses nicht unnötig zu stören. Das erste Lager ist in einem bevorzugten Ausführungsbeispiel durch ein zylindrisches Rundelement, welches durch Teile des Festlegeteils, als auch durch Teile des ersten Lagers hindurchgreift, fest und trotzdem teil - schwenkbar mit dem Festlegeteil verbunden. In gleicher Weise ist das zweite Lager durch ein entsprechend gestaltetes zylindrisches Rundelement, welches durch Teile des Grundelementes und des zweiten Lagers hineingreift teil - schwenkbar ausgeformt. Vorteilhaft am ersten und zweiten Lager ist der Umstand, dass ein optimales Zusammenspiel zwischen der Federkonstruktion als Zwischenstück zwischen dem Festlegeteil und dem Grundelement erreicht wird, wobei die Funktion des Kniehebelverschlusses positiv beeinflusst wird. Positiv bedeutet hierbei, dass der Nutzer sich auf eine fehlerfreie funktionierende Vorrichtung verlassen kann.

Gemäß der Erfindung sind das Festlegeteil und das Grundelement über ein Linearprofil verbunden. Es ist derart gestaltet, dass das Linearprofil an einem ersten Gelenk mit dem Festlegeteil und mit einem zweiten Gelenk am Grundelement verbunden ist. Als Gelenk ist hier wiederum ein Aufbau derart bezeichnet, bei der ein zylindrisches Rundelement jeweils durch Teile des Festlegeteils und des Linearprofils bzw. des Grundelements und des Linearprofils greifen. Ein Hindurchgreifen ist nicht zwingend notwendig. In ersten Linie soll das Linearprofil eine Verbindung zwischen dem Festlegeteil und dem Grundelement darstellen. Hierbei soll es nach Möglichkeit voll oder teil - schwenkbar mit dem jeweiligen Festlegeteil oder Grundelement verbunden sind. Vorteilhaft hierbei ist der Umstand, dass das Linearprofil durch die Bewegung des Kniehebelverschlusses zur Befestigung der Vorrichtung nach Möglichkeit unter dem Profil des Operationstisches in Richtung des Festlegeteils und gleichzeitig weg von der Zuordnung bewegt wird. Nur hierdurch wird eine entsprechende Festlegung der Vorrichtung an dem Profil des Operationstisches erreicht. Die Bewegung des Linearprofils hin zum Festlegeteil und weg von der Zuordnung leitet letztendlich den Befestigungsvorgang ein. Vorteilhaft hierbei ist ein einfacher Aufbau der entsprechend wenige Fehlerquellen aufweist und eine einfache Sterilisierung ermöglicht.

Die erfindungsgemäße Vorrichtung; ist derart gestaltet, dass das Linearprofil ein drittes Gelenk mit einem schwenkbar angeordneten Maulteil umfasst. Das dritte Gelenk ist derart gestaltet, dass zumindest eine Teil - Schwenkbewegung ermöglicht ist. Sie dient dazu, dass das Maulteil das Profil des Operationstisches entsprechend angreift und ein sicheres Festlegen erst ermöglicht.

Das Maulteil weist eine Eckkante auf, welche einen Teil des Profils des Operationstisches umfassen und halten kann. Durch die Kniehebel-Bewegung des Festlegeteil wird das Linearprofil weg von der Zuordnung hin zum Festlegeteil bewegt. Dabei wiederum bewegt das Linearprofil das Maulteil hin zu dem Profil des Operationstisches, so dass die Eckkante des Maulteil eine offene Kante des Profils anfährt, wobei durch die Befestigung der Vorrichtung durch Umlegen des Kniehebelverschlusses das Maulteil formschlüssig an einen Teilbereich des Profils angelegt und festgezogen wird. In diesem Zusammenhang ermöglicht es wiederum die Federkonstruktion, dass Operationstücher, welche um das Profil des Operationstisches gelegt wurden, das Maulteil nicht weiter beeinträchtigen. Die Federkonstruktion führt hier wiederum zu dem Umstand, dass für den Nutzer kein Unterschied spürbar ist, ob nun kein Tuch um das Profil des Operationstisches gelegt wurde oder bis zu drei Operationstücher um das Profil des Operationstisches gelegt wurden. Der Toleranzausgleich findet wie oben beschrieben durch die Federkonstruktion statt. Vorteilhaft hierbei ist der Umstand, dass eine sichere und für den Nutzer einfache Form des Festlegen der Vorrichtung durch Zusammenspiel des Grundelementes und des Maulteils erreicht wird.

Die erfindungsgemäße Vorrichtung; weist ein Maulteil auf, das eine Form angepasste bewegliche Wölbung umfasst, wobei diese Wölbung mit einer anderen Wölbung des Grundteils zusammenwirkt. Der Hintergrund hierbei ist der Umstand, dass bei der Relativbewegung des Maulteils hin zum Profil des Operationstisches je nach Lage des Grundelements oder der Anzahl der zwischen dem Profil des Operationstisches und der Vorrichtung gelegten Tücher die Eckkante des Maulteils möglichst formschlüssig mit der vorgesehenen Kante des Profils des Operationstisches schliessen soll. Während des Befestigens wird das Linearprofil weg von der Zuordnung gezogen. Dabei wird das Maulteil entlang der formangepassten Wölbung im Zusammenspiel mit der Wölbung des Grundelementes und durch eine Teil - Schwenkbewegung des Maulteils um das dritte Gelenk möglichst passgenau an die vorgesehene Kante des Profils des Operationstisches herangefahren. Vorteilig ist, dass das Maulteil durch eine simple konstruktive Lösung auf verschiedene Umstände, welche mit dem Profil des Operationstisches zusammenhängen angepasst werden kann.

Die formangepasste bewegliche Wölbung ist hierbei nicht besonders definiert. Lediglich das Zusammenspiel der formangepassten beweglichen Wölbung des Maulteils mit der anderen Wölbung des Grundteils ist von Belang. In welcher Weise die Wölbung gestaltet ist, ist unbeachtlich, solange das Ziel einer Entlangführung des Maulteils um die andere Wölbung des Grundteils ermöglicht wird.

Ein Ausführungsbeispiel der erfindungsgemässen Vorrichtung weist ausserdem einen Kniehebel auf, welcher am Festlegeteil ausgeformt ist. Dieser Kniehebel dient der einfachen Überwindung der erforderlichen Kraftspitze und soll dem Nutzer der erfindungsgemässen Vorrichtung durch Einsatz eines Hebels das Befestigen der Vorrichtung am Profil des Operationstisches erleichtern. Die ansonsten notwendige Kraft zum Schliessen der Vorrichtung wäre nicht ohne Weiteres aufzubringen. In diesem Zusammenhang ist der Kniehebel derart gestaltet, dass er auf der Aussenfläche der Zuordnung anliegt.

Hierbei soll vorteilhaft vermieden werden, dass bspw. abstehende Hebel für den Operateur ein Hindernis darstellen oder womöglich versehentlich wie ein Haken wirken.

Eine mit der Erfindung nutzbare Operationshilfe ist derart gestaltet, dass die Operationshilfe aus verschiedenen Teilen modulartig aufgebaut ist. So ist bspw. ein Ausführungsbeispiel derart ausgebildet, dass ein Zwischenstück vorhanden ist. Dieses Zwischenstück besteht aus einer Zahnstange und einem Verbindungselement, welche fest miteinander verbunden sind. Das Verbindungselement ist hierbei auf die Form der Zuordnung angepasst. In einem besonders bevorzugten Ausführungsbeispiel stellt die Zuordnung einen zylindrischen Grundkörper und das Verbindungselement stellt eine entsprechende Hülse dar, welche auf dem zylindrischen Grundkörper aufgefahren werden kann. Das Verbindungselement in Form einer Hülse hat den Vorteil, dass beim Drehen der Operationshilfe um die Zuordnung automatisch auch das Zwischenstück bestehend aus der Zahnstange und dem Verbindungselement um die Zuordnung herum gedreht werden kann. Hierbei würde sich auch das Zwischenstück als Teil der Operationshilfe um die Zuordnung drehen lassen.

Eine mir der Erfindung nutzbare Operationshilfe weist ausserdem einen Lagewechsler auf. Dieser Lagewechsler hat die Eigenschaft, dass er mit dem Zwischenstück und der Zuordnung vertikal verfahrbar und/oder 360° um die Zuordnung drehbar angeordnet ist. Der Lagewechsler kann hier entweder durch einen gestuften Justierer und/oder einen nicht gestuften Einsteller betätigt werden. Der Hintergrund ist hierbei, dass der Justierer aus einer Spindelanordnung besteht, deren erstes Ende mit der Zahnstange zusammenwirkt und deren zweites Ende einen Verstellkopf aufweist, welcher geeignet ist zur gestuften Justierung.

Das bedeutet im Einzelnen, dass durch die Spindelanordnung des Justierers entsprechend der Zahnstangen-Zähne und deren Abstand zueinander vertikal den gesamten Lagewechsler verfahren kann. Hierbei ist je nach dem in welche Richtung der Verstellkopf gedreht wird, ein vertikales Verschieben weg von dem Grundelement oder hin zum Grundelement möglich. Hierbei läuft ein Gewinde an den Zahnstangen-Zähnen entlang. Ein Drehen des Verstellkopfs ist entweder in Uhrzeigerrichtung oder entgegen der Uhrzeigerrichtung möglich. Allerdings ist der Verstellkopf nur als ein Beispiel zu betrachten. Hier ist auch eine andere Konstruktion denkbar. Lediglich der Umstand eines an den Zahnstangen vertikal bewegten Lagewechslers ist Zentrum der erfindungsgemässen Idee. Folglich werde bspw. auch eine elektrischer Antrieb denkbar. Weiter weist der Lagewechsler den nicht gestuften Einsteller auf. Dieser Einsteller stellt sich in einem bevorzugten Ausführungsbeispiel als Hebel dar, welcher dazu dient die Spindelanordnung des Justierers aus dem Zahnstangen-Eingriff zu bringen. Dadurch kann der Nutzer, welcher den Hebel des Einstellers betätigt den gesamten Lagewechsler vertikal verschieden, sobald er den Hebel in die ursprüngliche Position bringt, greift die Spindelanordnung des Justierers wieder in die vorgegebene Zahnstange ein und verrastet die Vertikal - Bewegung. Neben dem Lagewechsler, welcher eine Vertikalverstellung ermöglicht, umfasst die erfindungsgemässe Operationshilfe auch einen Horizontalversteller, der entlang der Spindelanordnung verschiebbar und festlegbar ist. Dieser Horizontalversteller kann durch ein entsprechendes Maulschlossprinzip auf einem Teil der Oberfläche des Justierers festgelegt und freigegeben werden. Hierzu dient der Einfachheit halber wieder ein Verstellkopf. Andere technische Lösungen, wie eine Flügelmutterschraube oder eine Federklammer kommen ebenso in Betracht. Für die Erfindungsidee ist von Belang, dass der Horizontalversteller entlang der Aussenfläche des Justierers, insbesondere der Aussenfläche der Spindelanordnung verschiebbar und festlegbar ist. Vorteilhaft hierbei ist eine einfache Nutzung und keine Fehlerquellen aufweisende Ausbildung des Horizontalverstellers. Der Operateur/Nutzer kann mit einer Hand den Horizontalversteller verschieben und festlegen. Solche ein Horizontalversteller ist bspw. notwendig, wenn zur Teilresektion eines inneren Organs bspw. der Brustkorb teilweise geöffnet und bei einem auf dem Rücken liegenden Patienten der Brustkorb angehoben werden muss. Dann kann der Operateur eine entsprechende Vertikalverstellung durch den Lagewechsler vornehmen, um zunächst die Höhe des vorhandenen Schnitts zu finden. Anschliessend kann er dann durch den Vertikalversteller die eine oder die andere Seite des Brustkorbs anfahren und eine sogenannte Valve in die Öffnung des Brustkorbs einbringen. Diese Valve hat eine grosse Auflagefläche und dient dazu an den Brustkorb von Innen herangefahren zu werden, um am Ende den Brustkorb zu heben. Das Heben wiederum kann der Operateur durch den Justierer des Lagewechslers vornehmen. Vorteilhaft ist hierbei, dass der Nutzer regelmässig nur eine Hand benötigt, um die Operationshilfe zu betätigen. Nachdem er sie betätigt hat, verbleibt sie ohne Weiteres zutun des Operateurs an der gewünschten Position.

Ein anderes Beispiel einer Operationshilfe weist eine Aufnahme für die Valve auf. Diese Aufnahme ist nicht leitend. Das bedeutet im Einzelnen, dass sie stromisoliert ist. Dies hat für den Nutzer zum Einen den Vorteil, dass bspw. bei Koagulation im Innern des Brustkorbs keine Stromübertragung auf den Horizontalversteller und damit auf die gesamte Vorrichtung bzw. den Tisch mehr erfolgen kann. Ausserdem ist die Aufnahme derart gestaltet, dass verschiedene Valven ohne Weiteres aufgenommen werden können. Zur stromisolierten Ausführung der Aufnahme kommen verschiedene Materialien, wie Kunststoff, Gummi od. dgl. in Betracht.

Eine Valve zum Einsetzen in die Aufnahme des Horizontalverstellers kann eine Absaugeinrichtung umfassen. Diese Absaugeinrichtung ist in einem bevorzugten Ausführungsbeispiel derart ausgebildet, dass ein Teilbereich der Valve eine Öffnung aufweist, welche über Leitungen mit einer Unterdruckquelle verbunden werden können. Dies hat zur Folge, dass bspw. bei der Koagulation entstehender Rauch durch die Öffnung an der Valve abgezogen und aus dem Körper rausgeleitet wird, was wiederum zu schnelleren Genesungszeiten des Patienten führt.

### FIGURENBESCHREIBUNG

Im Folgenden wird ein bevorzugtes Ausführungsbeispiel der Erfindung anhand einiger schematischer Zeichnungen beschrieben, wobei die Zeichnungen zeigen:
**Figur 1** eine Teil-Seitenansicht einer erfindungsgemässen Vorrichtung, teilweise durchsichtig;
**Figur 2** eine andere Teil-Seitenansicht der Figur 1 von schräg oben;
**Figur 3** eine Teil-Seitenansicht nach Figur 1;
**Figur 4** eine Teil-Seitenansicht der Vorrichtung nach Figur 1 von schräg oben in befestigter Lag;
**Figur 5** eine andere Teil-Seitenansicht der Figur 4, teilweise durchsichtig;
**Figur 6** eine schematische Ansicht der erfindungsgemässen Vorrichtung;
**Figur 7** eine schematische Draufsicht auf einen Teil der erfindungsgemässen Vorrichtung;
**Figur 8** eine teilweise durchsichtige Seitenansicht der Figur 7;
**Figur 9** eine perspektivische Teildarstellung einer alternativen, nicht erfindungsgemäßen Vorrichtung in einem geöffeneten Zustand;
**Figur 10**eine perspektivische Teildarstellung der Vorrichtung nach Figur 9 in einem geschlossenen Zustand;
**figur 11** eine perspektivische Darstellung einer Valve,

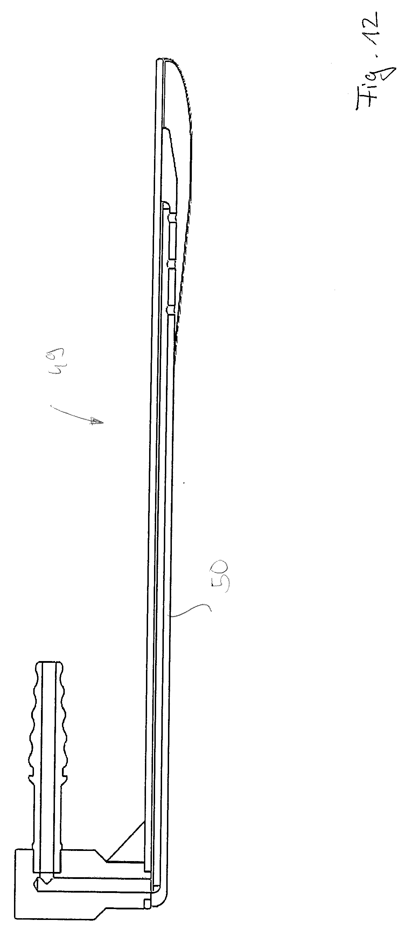

**Figur 12** eine Schnittdarstellung der Valve nach Figur 11.

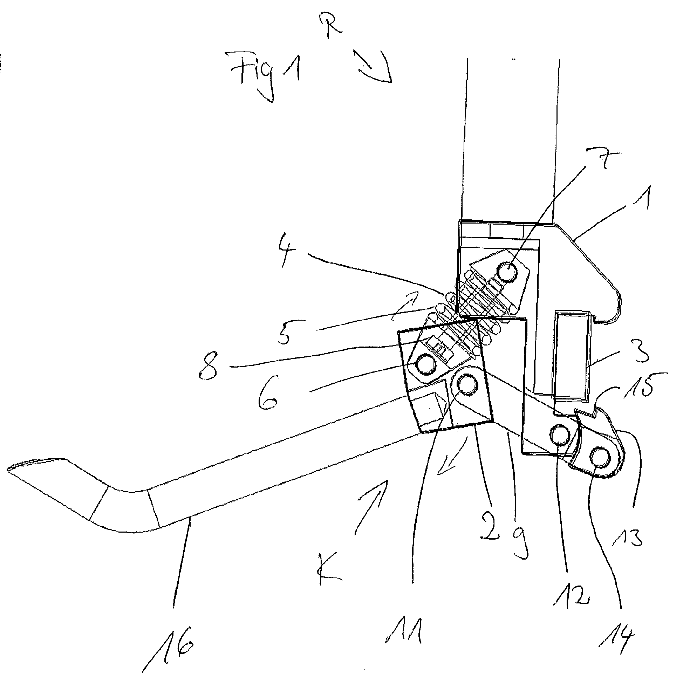

In der Figur 1 ist eine erfindungsgemässe Vorrichtung R zur Befestigung an einem Operationstisch gezeigt. Die Vorrichtung besteht aus einem Grundelement 1 und einem Festlegeteil 2. Vom Operationstisch ist lediglich das Profil 3 gezeigt, welches ähnlich einer Rehling durch Stege vom Operationstisch beabstandet angebracht ist oder in anderer Weise teilweise mit dem Rest des Operationstisches verbunden ist. Das Profil 3 stellt sich regelmässig als Teil des Operationstisches dar.

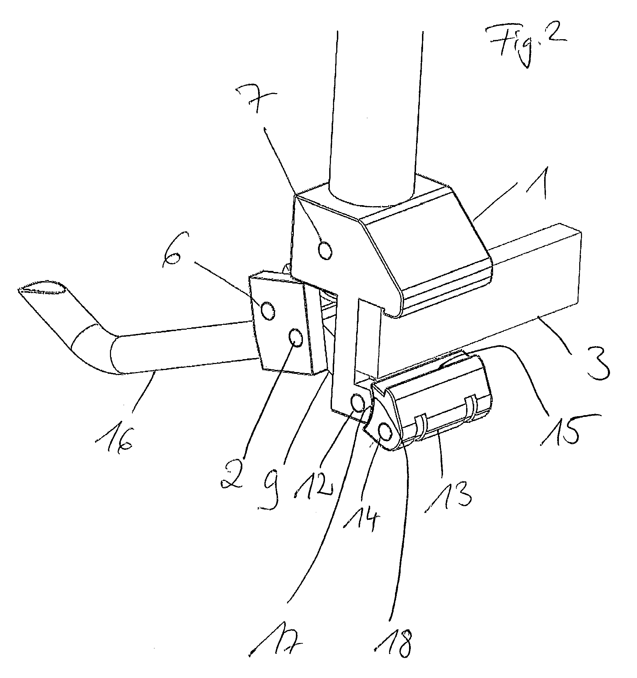

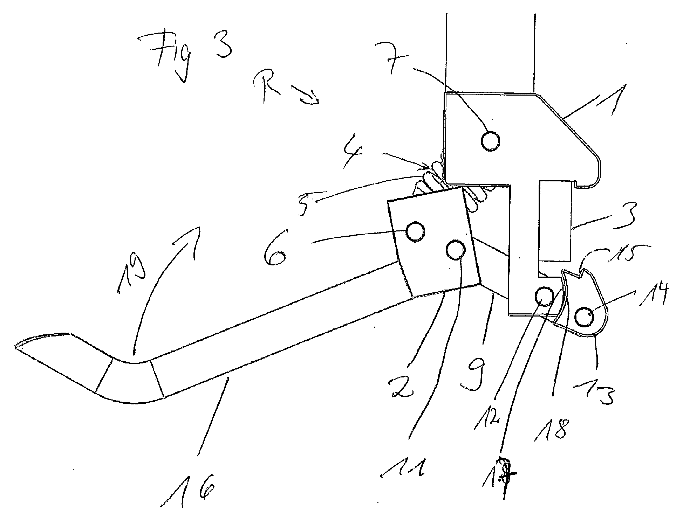

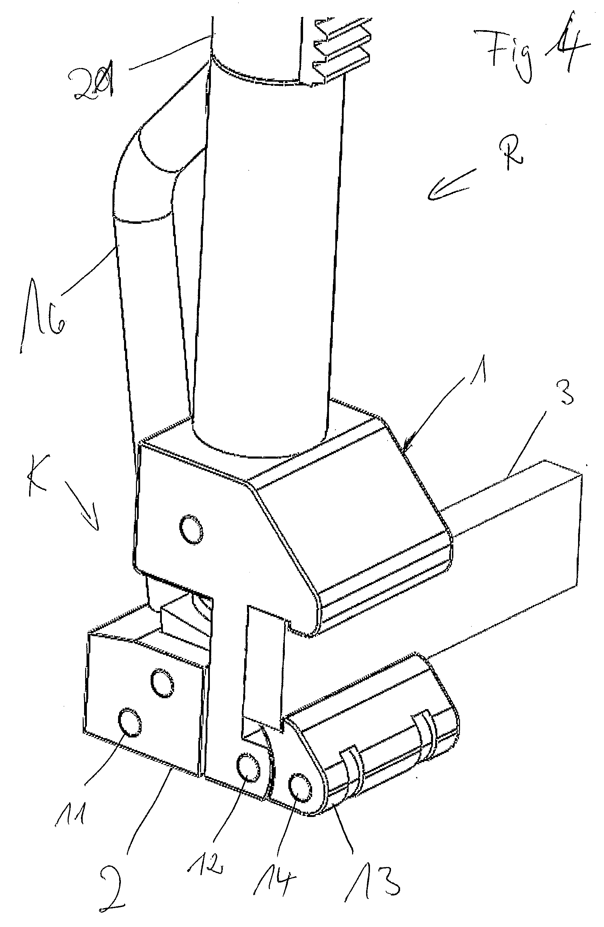

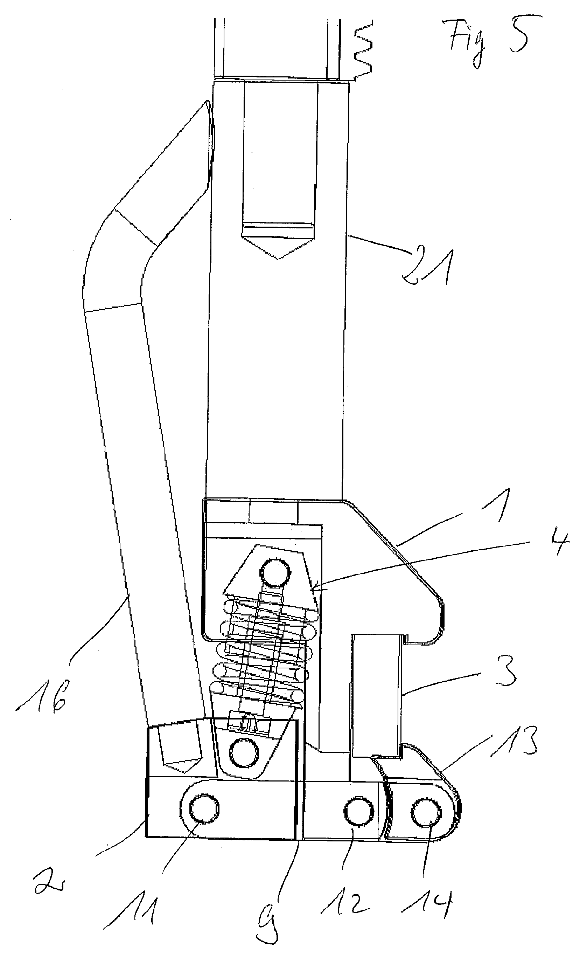

Ausserdem ist in Figur 1 der Kniehebelverschluss K zu sehen. Das Grundelement 1 und das Festlegeteil 2 stellen sich als Teil des Kniehebelverschlusses K dar. Ausserdem ist Teil des Kniehebelverschlusses eine Federkonstruktion 4. Diese Federkonstruktion 4 besteht aus einem vorgespannten Federelement 5, welches zwischen einem ersten Lager 6 und einem zweiten Lager 7 angeordnet ist. Das erste Lager 6 und das zweite Lager 7 werden durch eine Schraube 8 miteinander verbunden, wobei die Schraube 8 das Federelement 5 vollständig durchgreift. In der Seitenansicht der Figur 1 ist ausserdem gut zu erkennen, dass das Festlegeteil 2 und das Grundelement 1 durch eine Linearprofil 9 miteinander verbunden sind. Das Linearprofil 9 weist hierzu ein erstes Gelenk 11 und ein zweites Gelenk 12 auf, wobei das Linearprofil 9 durch das erste Gelenk 11 mit dem Festlegeteil 2 drehbar, schwenkbar verbunden ist und über das zweite Gelenk 12 mit dem Grundelement 1 drehbar, verschwenkbar verbunden ist. Ausserdem ist ein Maulteil 13 zu erkennen, welches über ein drittes Gelenk 14 mit dem Linearprofil 9 wiederum drehbar, schwenkbar verbunden ist. Das Maulteil 13 weist eine Eckkante 15 auf, welche eine bestimmte Kante des Profils 3 angreifen soll. Ausserdem ist in Figur 1 gut zu erkennen, wie das Festlegeteil 2 einen Kniehebel 16 ausgebildet. Insgesamt ist in Figur 1 eine teilweise geschnittene Seitenansicht gezeigt, bei der der Kniehebelverschluss geöffnet ist, so dass die Vorrichtung R nicht an dem Profil 3 festangelegt ist. In gleicher Weise ist eine andere Ansicht in Figur 2 zu erkennen. In diesem Zusammenhang wird darauf hingewiesen, dass sich in den Figuren wiederholende Bezugsziffern nicht nochmals beschrieben werden. Vielmehr soll klargestellt werden, dass Merkmale mit gleichen Bezugsziffern auch die gleiche Bedeutung wie in den vorbeschriebenen Figuren haben. In Figur 2 ist gut zu erkennen., wie das Maulteil 13 über die Dreh-Schwenkachse, das dritte Gelenk an einer Wölbung 17 des Grundteils 1 entlanggefahren wird, wobei das Maulteil 13 eine entsprechende Wölbung 18 aufweist, damit ein Entlangfahren problemlos möglich ist. Figur 3 zeigt nochmals die aus Figur 1 geschnitten gezeigte Ansicht in nicht geschnittener Darstellung. Auch dort ist nochmals gut zu erkennen, wie die Wölbung 17 und die Wölbung 18 miteinander korrespondieren, um ein Entlanggleiten des Maulteils 13 an der Wölbung 17 des Grundteils 1 zu ermöglichen. In Figur 3 ist ausserdem ein Pfeil 19 gezeigt, welcher die Richtung der Bewegung des Kniehebels 16 zum Befestigen der Vorrichtung R an dem Profil 3 andeutet. Bei Bewegung des Kniehebels 16 entlang de Pfeils 19 hin zum Grundteil 1 wird das Festlegeteil 2 derart verlagert, dass sich das erste Gelenk 11 vom zweiten Lager 7 fortbewegt. Durch diese Fortbewegung wird wiederum das Maulteil 13 entlang der Wölbung 17 an das Profil 3 herangefahren, da das Profil 9 aus einer senkrechten Position in eine waagrechte Position verbracht wird. Dadurch entsteht um das zweite Gelenk 12 eine Drehbewegung des Maulteils 13 hin zum Profil 3. In Figur 4 ist nun eine schematische Ansicht eines geschlossenen Kniehebelverschlusses K zu erkennen. Dort liegt der Kniehebel 16 an einer Zuordnung 20 an. Das Festlegeteil 2 ist in Figur 4 direkt an einen Bereich des Grundteils 1 herangefahren und das nicht gezeigte Linearprofil 9 liegt nun im wesentlichen im 90° Winkel zum Profil 3. Das Maulteil 13 hat in Figur 4 die vorgesehene Kante des Profils 3 angegriffen und festgelegt. Das erste Gelenk 11, das zweite Gelenk 12 und das dritte Gelenk liegen in Figur 4 in einer Ebene, was wiederum bestätigt, dass das nicht gezeigte Linearprofil 9 in etwa rechtwinklig zum Profil 3 ausgerichtet ist. In Figur 5 ist eine geschnittene Seitenansicht der Figur 4 zu sehen. Dort ist im Einzelnen nochmals gut zu erkennen, wie das Linearprofil 9 in eine Klemmposition gebracht wurde. Der Kniehebel 16 liegt in dieser Position an einer Zuordnung 21 an. Daneben ist in Figur 5 gut zu erkennen, wie die Federkonstruktion 4 nun im wesentlichen parallel zum Profil 3 gelagert ist. Dies ist dadurch entstanden, dass durch die Bewegung des Kniehebels 16 in Richtung des Pfeils 19 das erste Lager 6 hin zum Profil 3 gedrückt wurde. In Figur 1 ist die Federkonstruktion 4 noch in einem Winkel > 30° vom Profil 3 abgewandt. In Figur 5 ist dies aufgehoben.

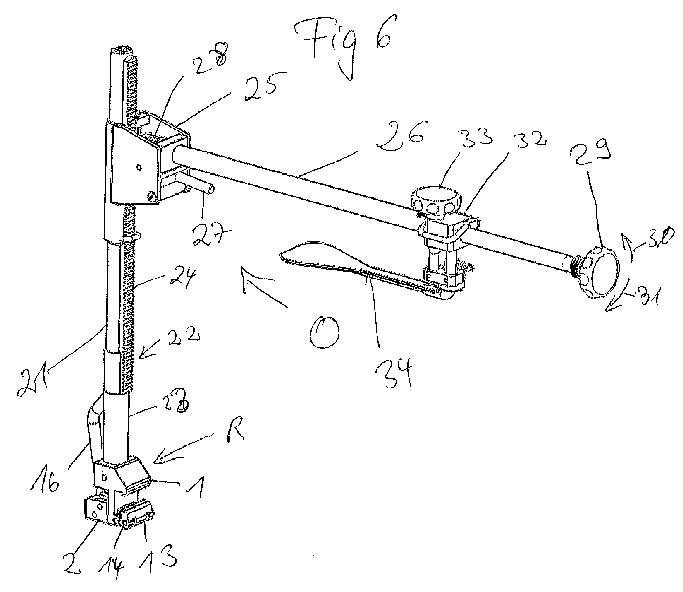

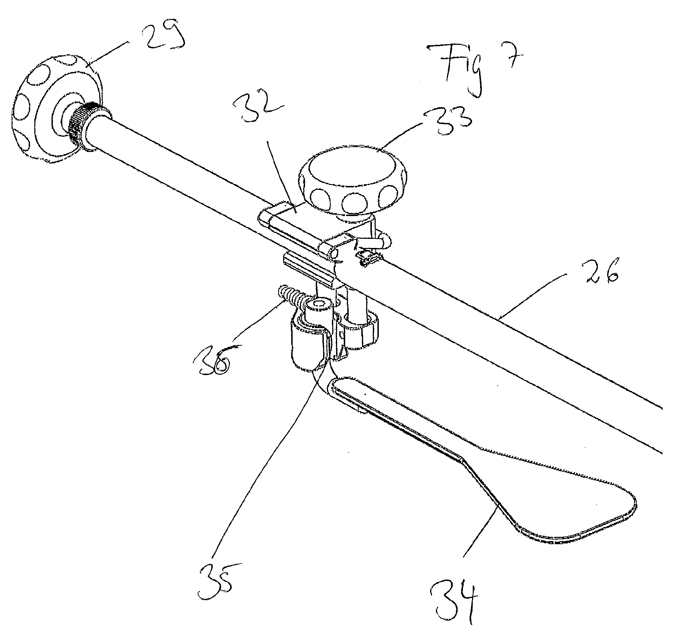

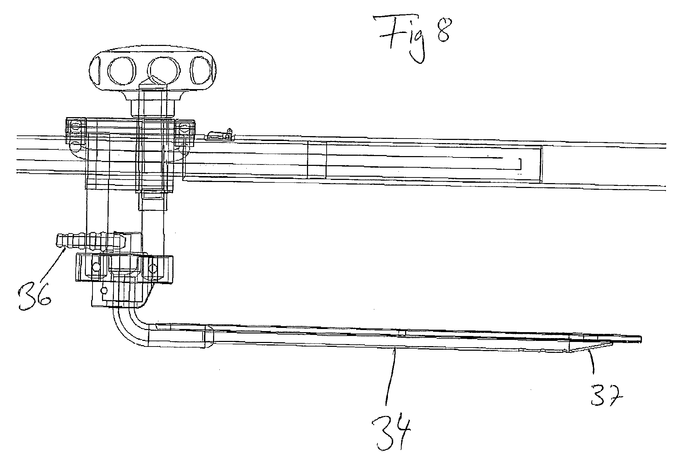

Figur 6 zeigt neben der Vorrichtung R, eine Operationshilfe O. Diese Operationshilfe besteht unter anderem aus einem Zwischenstück 22, welches wiederum aus einem Verbindungselement 23 und einer Zahnstange 24 gebildet ist und einem Lagewechsler 25, welcher wiederum einen Justierer 26 und einen Einsteller 27 aufweist. Der Justierer 26 besteht aus einem nicht gezeigten Spindelanordnung deren Kopf 28 mit der Zahnstange 24 zusammenwirkt. Ausserdem ist aus dem Kopf 28 abgewandten Ende des Justierers 26 ein Verstellkopf 29 gezeigt, welcher durch Drehen, wie sie in den Pfeilen 30 und 31 angedeutet sind mit Hilfe der Spindelanordnung den Lagewechsler 25 entlang der Zahnstange hin zur Vorrichtung R oder weg von der Vorrichtung R bewegen kann. Ausserdem ist ein Horizontalversteller 32 mit einem Verstellkopf 33 zu erkennen, welcher auf der Aussenfläche des Justierers 26 bewegt, verschoben und festgelegt werden kann. Die Festlegung erfolgt in der Regel durch entsprechendes Betätigen eines Verstellkopfes 33. Daneben ist auch eine Valve 34 zu erkennen, welche in eine Aufnahme 35, wie sie in Figur 7 gezeigt ist, gelagert. Diese Valve 34 weist einen Anschluss 35 auf, welcher mit einer Unterdruckquelle verbunden ist, welche hier nicht gezeigt ist. In Figur 8 ist nochmals die Valve 34 in geschnittener Seitenansicht zu erkennen. Dort ist eine Öffnung 37 auf der Unterseite der Valve 34 gezeigt, welche über ein Kanalsystem zu der Anschlussstelle 36 verläuft. Auf diese Weise kann eine nicht gezeigte Unterdruckquelle an den Anschluss 36 angeschlossen werden, was wiederum dazu führen würde, dass durch die Öffnung 37 Teile durch den Anschluss 36 gezogen werden würden. Der Anschluss 36 und die Öffnung 37 stellen eine Absaugeinrichtung dar.

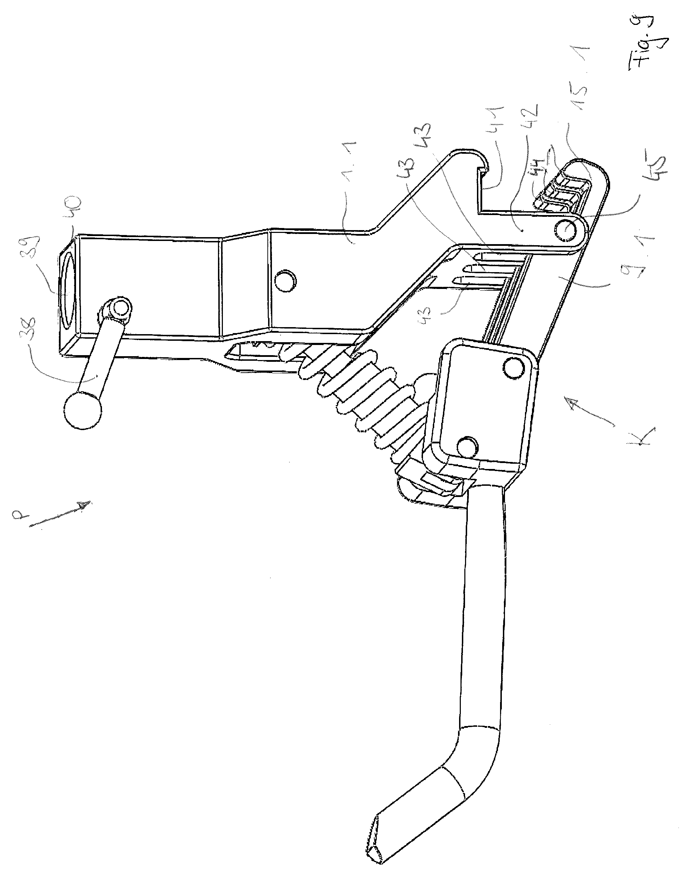

Figur 9 zeigt einen Teil eines alternativen, nicht erfindungsgemäßen Beispiels einer Vorrichtung. Im wesentlichen ist die Vorrichtung P analog zu der Vorrichtung R der Figur 1 ausgebildet. Die Vorrichtung P umfasst ebenfalls einen Kniehebelverschluss K. Dazu wird auf die Beschreibung in Figur 1 verwiesen.

Ein Grundelement 1.1 und ein Linearprofil 9.1 sind anders als bei der Vorrichtung R der Figur 1 ausgebildet.

Das Grundelement 1.1 umfasst eine Feststelleinrichtung 38 und eine Öffnung 39 an ihrer Oberseite 40. In die Öffnung 39 kann einen nicht dargestellte Zuordnung eingebracht werden über die Feststelleinrichtung 38 kann die nicht dargestellte Zuordnung in der Öffnung 39 festgelegt werden. Dadurch ergibt sich der Vorteil, dass die Zuordnung je nach Anwendungszweck passend verwendet werden kann. Vorzugsweise können Zuordnungen mit unterschiedlichen Längen, Zahnstangen, Anschlusselementen, Rastschienen, Steckelementen, Löchern und/oder Biegungen eingesetzt werden. Besonders bevorzugt weist die Zuordnung eine Fläche auf, die sich zum Klemmen mit der Feststelleinrichtung eignet. Vorzugsweise ist diese Fläche an der Seite der Zuordnung ausgebildet, die dem zu klemmenden Profil abgewandt ist.

Das Grundelement 1.1 weist einen oberen Anschlag 41 auf. An den oberen Anschlag 41 schließt vorzugsweise rechtwinklig, ein seitlicher Anschlag 42 an. In der seitliche Anschlag 42 ist vorzugsweise einstückig mit dem Grundelement 1.1 ausgebildet. Des weiteren weist der seitliche Anschlag 42 eine derartige Fläche auf. Dadurch ist das Grundelement 1.1 sehr einfach herstellbar.

Das Linearelemente 9.1 weist an seinem vorderen Ende eine Eckkante 15. 1 auf. Die Eckkante 15.1 und das Linearelement 9.1 sind aus einem Stück gebildet.

Das Linearelement 9.1 weist eine Mehrzahl von Stegen 44 auf.

Das Linearelement 9.1 und das Grundelement 1.1 beziehungsweise der seitliche Anschlag 42 sind über ein Gelenk 45 miteinander verbunden. Die Stege 44 des Linearelements 9.1 greifen dabei durch die Spalten 43 des seitlichen Anschlags 42.

Die Vorrichtung P hat gegenüber der Vorrichtung R den Vorteil, dass sie ein Gelenk weniger aufweist, dadurch einfache herstellbar ist, besser zu reinigen und stabiler ist.

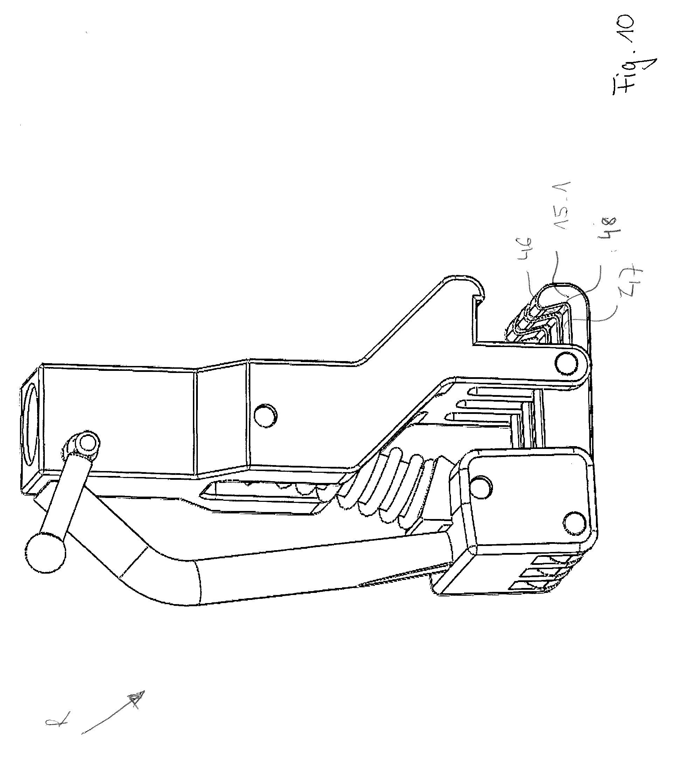

Figur 10 zeigt die Vorrichtung R in einem geschlossenen Zustand. Hier ist erkennbar, dass ein äußerer Anschlag 46 der Eckkante 15.1 gegen über einen unteren Anschlag 47 der Eckkante 15.1 einem Winkel kleiner 90° einschließt dadurch ergibt sich der Vorteil, dass ein nicht dargestellte Profil eine Ecke 48 zwischen dem äußeren Anschlag 46 dem unteren Anschlag 47 nicht berührt. Es ergibt sich sozusagen ein Freischnitt. Dadurch ergibt sich der Vorteil, dass die Vorrichtung P sowohl an Profilen ohne Operationstuch, wie auch Profilen mit Operation sicher festgelegt werden kann.

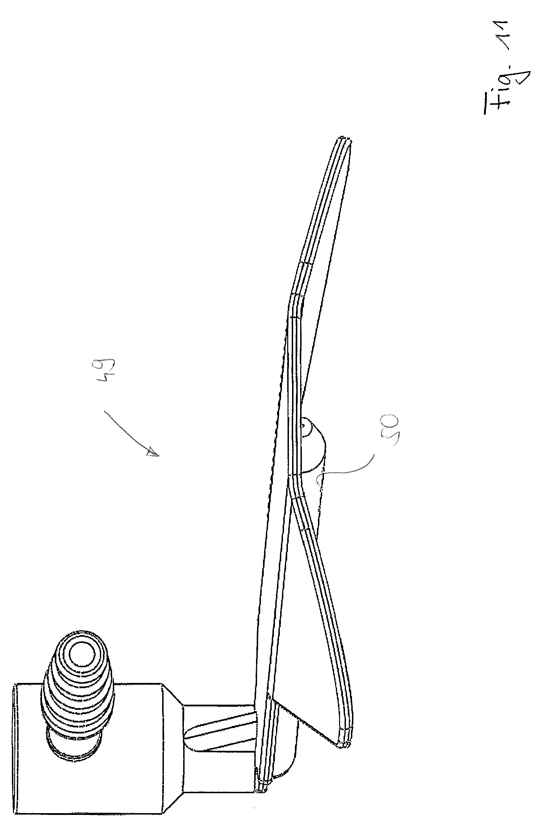

Figur 11 zeigt ein Beispiel einer Valve 49. Die Valve 49 ist vorzugsweise aus einem 1,5 mm dicken Blech, vorzugsweise Federstahl hergestellt Dadurch ist die Valve 49 sehr steif, und ermöglicht eine bessere Druckverteilung. Durch die Verwendung von Federstahl kann die verwendete Materialstärke von 3 mm auf 1,5 mm reduziert werden.

### Bezugszeichenliste

| | | | | | |
|---|---|---|---|---|---|
| 1 | Grundelement | 34 | Valve | 67 | |
| 2 | Festlegteil | 35 | Aufnahme | 68 | |
| 3 | Profil | 36 | Anschluss | 69 | |
| 4 | Federkonstruktion | 37 | Öffnung | 70 | |
| 5 | Federelement | 38 | Feststelleinrichtung | 71 | |
| 6 | Erstes Lager | 39 | Öffnung | 72 | |
| 7 | Zweites Lager | 40 | Oberseite | 73 | |
| 8 | Schraube | 41 | Oberer Anschlag | 74 | |
| 9 | Linearprofil | 42 | Seitlicher Anschlag | 75 | |
| 10 | | 43 | Spalt | 76 | |
| 11 | Erstes Gelenk | 44 | Steg | 77 | |
| 12 | Zweites Gelenk | 45 | Gelenk | 78 | |
| 13 | Maulteil | 46 | Äusserer Anschlag | 79 | |
| 14 | Drittes Gelenk | 47 | Unterer Anschlag | | |
| 15 | Eckkante | 48 | Ecke | K | Kniehebelverschluss |
| 16 | Kniehebel | 49 | Valve | O | Operationshilfe |
| 17 | Wölbung | 50 | Absaugrohr | P | Vorrichtung |
| 18 | Wölbung | 51 | | R | Vorrichtung |
| 19 | Bewegungsteil | 52 | | | |
| 20 | | 53 | | | |
| 21 | Zuordnung | 54 | | | |
| 22 | Zwischenstück | 55 | | | |
| 23 | Verbindungselement | 56 | | | |
| 24 | Zahnstange | 57 | | | |
| 25 | Lagewechsler | 58 | | | |
| 26 | Justierer | 59 | | | |
| 27 | Einsteller | 60 | | | |
| 28 | Kopf | 61 | | | |
| 29 | Verstellkopf | 62 | | | |
| 30 | Richtungspfeil | 63 | | | |
| 31 | Richtungspfeil | 64 | | | |
| 32 | Horizontalversteller | 65 | | | |
| 33 | Verstellkopf | 66 | | | |

## Patentansprüche

1. Vorrichtung zur Befestigung an einem Linearprofil (3) eines Operationstisches, mit einem Grundelement (1), einem Festlegeteil (2), einem Maulteil (13) und einem Kniehebelverschluss (K)
**dadurch gekennzeichnet,**
**dass** das Festlegeteil (2) und das Grundelement (1) über ein Linearprofil (9) an einem ersten Gelenk (11) und einem zweiten Gelenk (12) wirkverbunden sind und dieses wirkverbundene Linearprofil (9) ein drittes Gelenk (14) umfasst, an dem das Maulteil (13) schwenkbar angeordnet ist, wobei das Maulteil (13) eine formangepasste Wölbung (18) aufweist, welche mit einer anderen Wölbung (17) des Grundelements (1) so zusammenwirkt, dass das Maulteil der Wölbung des Grundteils entlang geführt wird.

2. Vorrichtung nach Anspruch 1, **dadurch gekennzeichnet, dass** der Kniehebelverschluss (K) eine Federkonstruktion (4) umfasst, geeignet zum Toleranzausgleich bei der Befestigung.

3. Vorrichtung nach Anspruch 2, **dadurch gekennzeichnet, dass** die Federkonstruktion (4) aus einem vorgespannten Federelement (5) zwischen einem ersten Lager (6) und einem zweiten Lager (7) besteht.

4. Vorrichtung nach Anspruch 3, **dadurch gekennzeichnet, dass** das Festlegeteil (2) und das Grundelement (1) durch die Federkonstruktion (4) über das erste Lager (6) und das zweite Lager (7) wirkverbunden sind.

5. Vorrichtung nach einem der Ansprüche 1 bis 4, **dadurch gekennzeichnet, dass** das Grundteil (1) eine Zuordnung (21) aufweist, geeignet zur Aufnahme einer Operationshilfe (O).

6. Vorrichtung nach einem der Ansprüche 1 bis 5, **dadurch gekennzeichnet, dass** das Festlegeteil (2) einen Kniehebel (16) ausbildet, wobei die dem Festlegeteil (2) abgewandte Seite eine angepasste Form, geeignet zum Anliegen auf einer Aussenfläche reiner Zuordnung (21) ausbildet.

## Claims

1. A device for fastening to a linear profile (3) of an operating table, with a base element (1), a fixing part (2), a jaw part (13) and a toggle lever closure (K),
**characterised in that**
the fixing part (2) and the base element (1) are in an operative connection via a linear profile (9) on a first articulation (11) and a second articulation (12), and this operatively-connected linear profile (9) comprises a third articulation (14) on which the jaw part (13) is pivotably arranged, the jaw part (13) having a shape-adapted curvature (18) which cooperates with another curvature (17) of the base element (1) such that the jaw is guided along part of the curvature of the base part.

2. A device according to Claim 1, **characterised in that** the toggle lever closure (K) comprises a spring structure (4) suitable for tolerance compensation upon fastening.

3. A device according to Claim 2, **characterised in that** the spring structure (4) consists of an initially tensioned spring element (5) between a first bearing (6) and a second bearing (7).

4. A device according to Claim 3, **characterised in that** the fixing part (2) and the base element (1) are in an operative connection owing to the spring structure (4) via the first bearing (6) and the second bearing (7).

5. A device according to one of Claims 1 to 4, **characterised in that** the base part (1) has an association means (21) suitable for receiving a surgical tool (O).

6. A device according to one of Claims 1 to 5, **characterised in that** the fixing part (2) forms a toggle lever (16), the side remote from the fixing part (2) forming an adapted shape, suitable for resting against an outer surface of an association means (21).

## Revendications

1. Dispositif de fixation à un profil linéaire (3) d'une table d'opération, avec un élément de base (1), un élément de serrage (2), un élément de mâchoire (13) et une genouillère (K),
**caractérisé par le fait**
**que** l'élément de serrage (2) et l'élément de base (1) sont connectés activement par l'intermédiaire d'un profil linéaire (9) à une première articulation (11) et à une deuxième articulation (12) et que ce profil linéaire (9) connecté activement comporte une troisième articulation (14) sur laquelle est disposé de manière pivotante l'élément de mâchoire (13), dans lequel l'élément de mâchoire (13) présente un bombage de forme adaptée (18) qui coopère avec un autre bombage (17) de l'élément de base (1) de sorte que l'élément de mâchoire soit guidé le long du bombage de l'élément de base.

2. Dispositif selon la revendication 1, **caractérisé par le fait que** la genouillère (K) comporte une structure de ressort (4) apte à compenser les tolérances lors de la fixation.

3. Dispositif selon la revendication 2, **caractérisé par le fait que** la structure de ressort (4) est constituée d'un élément de ressort précontraint (5) entre un premier palier (6) et un deuxième palier (7).

4. Dispositif selon la revendication 3, **caractérisé par le fait que** l'élément de serrage (2) et l'élément de base (1) sont reliés activement par la structure de ressort (4) par l'intermédiaire du premier palier (6) et du deuxième palier (7).

5. Dispositif selon l'une des revendications 1 à 4, **caractérisé par le fait que** l'élément de base (1) présente une association (21) convenant pour recevoir une aide d'opération (O).

6. Dispositif selon l'une des revendications 1 à 6, **caractérisé par le fait que** l'élément de serrage (2) constitue un levier à genouillère (16), le côté opposé à l'élément de serrage (2) constituant une forme adaptée convenant pour s'appuyer sur une face extérieure d'une association (21).
